# FASCICULE DE BREVET EUROPEEN

(11) **EP 3 086 853 B1**
(45) Date de publication et mention de la délivrance du brevet: **09.08.2023**
(21) Numéro de dépôt: 14833257.0
(22) Date de dépôt: 19.12.2014
(51) Int. Cl.: A61Q 1/02, A61K 8/02, B44C 1/17, A61B 5/00, B41J 3/407, A61Q 1/10, A45D 44/00

(54) **DISPOSITIF DE MAQUILLAGE COMPORTANT UNE PLURALITÉ D'ENCRES COSMÉTIQUES**
MAKEUPVORRICHTUNG MIT MEHREREN KOSMETISCHEN FARBSTOFFEN
MAKEUP DEVICE COMPRISING A PLURALITY OF COSMETIC DYES

(30) Priorité: 27.12.2013 FR 1363637
(43) Date de publication de la demande: 02.11.2016
(73) Titulaire: L'OREAL, 75008 Paris (FR)
(72) Inventeur: SAMAIN, Henri, 91570 Bièvres (FR); GIRON, Franck, 77400 Lagny Sur Marne (FR)
(74) Mandataire: Cabinet Nony
(86) Numéro de dépôt international: PCT/IB2014/067138
(87) Numéro de publication internationale: WO 2015/097620

(56) Documents cités:
- WO-A1-2010/004531
- JP-A- 2001 278 739
- JP-A- 2007 204 487
- JP-A- 2013 177 325
- US-B1- 6 190 730
- US-B2- 8 007 062
- Dyno Pretty Pup: "Dyno Pretty Pup Beauty Diary: LA Colors 30 Eye Design Palettes - Review", Dyno Pretty Pup Beauty Diary, 16 mars 2012 (2012-03-16), XP055134860, Extrait de l'Internet: URL:http://dynopupbeauty.blogspot.nl/2012/ 03/la-colors-30-eye-design-palettes-review .html [extrait le 2014-08-14]

## Description

La présente invention concerne un dispositif de maquillage portant une ou plusieurs encres colorantes déposées par impression.

### Arrière-plan

On connaît la réalisation de motifs cosmétiques sur la peau par usage de décalcomanies nécessitant l'ajout d'un liquide tiers afin d'obtenir un transfert du motif sur la peau.

D'autres dispositifs de maquillage comme des palettes regroupant une ou plusieurs compositions de maquillage différentes produisant des couleurs distinctes sont, par ailleurs, connus.

Toutefois, de tels dispositifs proposent à l'utilisateur une palette de couleurs relativement limitée et pas nécessairement adaptée au goût de l'utilisateur. De tels dispositifs limitent par conséquent les effets de maquillage pouvant être obtenus et peuvent ne pas permettre à l'utilisateur de personnaliser à loisir son maquillage. Ils peuvent ne pas être adaptés aux différents types de peau existants.

JP 2013 177325 A divulgue un procédé de préparation d'un ensemble cosmétique selon le préambule de la revendication 1.

Il existe un besoin pour bénéficier de nouveaux dispositifs de maquillage.

Il existe encore un besoin pour obtenir des dispositifs de maquillage permettant d'obtenir simplement des maquillages complexes et personnalisés.

L'invention vise à répondre à tout ou partie de ces besoins.

### Résumé

L'invention est définie par les caractéristiques de la revendication 1.

Selon un premier aspect, il est présenté un dispositif de maquillage, comportant un substrat définissant une surface d'impression présentant au moins une région, notamment une pluralité de régions, imprimée avec au moins une couche d'au moins une encre colorante destinée à être appliquée sur les matières kératiniques humaines, ladite au moins une couche d'encre colorante
- ayant été déposée par impression sur la surface d'impression par au moins une imprimante,
- n'étant pas recouverte par un adhésif, et
- l'encre colorante produisant après application et transfert sur les matières kératiniques au moins un effet optique visible parmi la couleur et/ou la brillance.

Le dispositif comporte par exemple une pluralité de régions, chacune des régions étant imprimée avec une couche d'encre colorante cosmétique différente, les encres colorantes étant destinées à être appliquées sur les matières kératiniques humaines, les couches d'encre ayant été déposées par impression sur le substrat par au moins une imprimante et n'étant pas recouvertes par un adhésif, chaque encre colorante produisant, après application sur les matières kératiniques, au moins un effet optique visible différent choisi parmi la couleur et la brillance .

Il est aussi présenté un dispositif de maquillage comportant un substrat présentant au moins une région, ou une pluralité de régions, chacune des régions portant une couche d'encre colorante cosmétique différente, les encres colorantes étant destinées à être appliquées sur les matières kératiniques humaines et étant aptes à produire un maquillage par application sur les matières kératiniques sans ajout d'un composé fluide tiers, chaque couche d'encre colorante ayant été déposée par impression sur le substrat par au moins une imprimante et produisant après application sur les matières kératiniques, au moins un effet optique visible différent, choisi parmi la couleur et la brillance.

L'invention permet notamment l'obtention de dispositifs de maquillage sous la forme de palettes préparées par impression d'encres colorantes cosmétiques.

La mise en oeuvre de couches d'encre colorante obtenues par impression à l'aide d'une imprimante permet avantageusement, par rapport aux dispositifs de maquillage classiques, d'obtenir un maquillage complexe et personnalisable.

Une couche peut comporter une ou plusieurs encres. Deux couches d'encre diffèrent par la nature chimique de la ou des encres qui les composent ou par les proportions relatives de chaque encre. L'invention permet à l'utilisateur de choisir sa ou ses couleurs, évitant ainsi d'être restreint à des choix de couleurs limités, voire inadaptés. La richesse des couleurs offerte à l'utilisateur, permise par l'impression, permet d'obtenir des maquillages produisant un effet particulièrement esthétique, et se rapprochant par exemple au mieux du teint naturel ou bronzé de l'utilisateur.

L'invention permet avantageusement à un utilisateur de créer un dispositif de maquillage personnalisé portant des couches d'encre colorante dont l'une au moins produit une couleur correspondant au teint de l'utilisateur, permettant ainsi d'atteindre un résultat de maquillage très naturel.

Tout ou partie des couches d'encre colorante peuvent comporter une ou plusieurs matières colorantes telles que décrites ci-après.

De préférence, tout ou partie des couches d'encre colorante présentes sur le dispositif ne sont pas entièrement sèches après une durée de 15 minutes après l'impression, notamment après un temps de 24 heures et mieux après une durée de 7 jours au contact avec l'air et hygrométrie normale (55 % d'humidité relative) et 20°C, sous pression atmosphérique.

L'application sur les matières kératiniques d'une encre colorante à partir d'une couche non entièrement sèche facilite le transfert de l'encre.

Tout ou partie des couches d'encre colorante peuvent être sous forme fluide lorsqu'elles sont portées par la surface d'impression du substrat immédiatement avant l'application sur les matières kératiniques. Les encres colorantes peuvent ne pas comporter de pigment et/ou de charge particulaire. Cela peut faciliter l'utilisation de la technologie d'impression numérique par jet d'encre.

Avantageusement, l'encre a une viscosité allant de 1mPa.s à 500 mPa.s, et de préférence de 1mPa.s à 300 mPa.s , à 25 °C.

La viscosité de l'encre peut être mesurée selon tout procédé connu de l'homme de l'art, et notamment selon le procédé conventionnel suivant. A 25 °C à l'aide d'un Rhéomat 180, équipé d'un mobile tournant à 200 tours/mn, l'homme du métier peut choisir le mobile permettant de mesurer la viscosité, parmi les mobiles, M1 ou M2 ou M3 ou M4 sur la base de ses connaissances générales, de manière à pouvoir réaliser la mesure.

Dans une variante, l'encre est déposée sous forme pulvérulente sur la surface d'impression, par exemple par une imprimante laser à four désactivé.

Dans un exemple de réalisation, une matière colorante est présente dans tout ou partie des encres colorantes, en une teneur massique allant de 0, 01 à 60 %, par rapport à la masse totale de l'encre; de préférence allant de 0,1 à 40 %; préférentiellement allant de 0,1 à 20 %. La matière colorante est préférentiellement constituée d'un ou plusieurs colorants.

Dans un exemple de réalisation, tout ou partie des encres colorantes comporte un solvant liquide, par exemple de l'eau, dans lequel la matière colorante est présente, le solvant liquide étant présent dans chacune des encres colorantes en une teneur massique allant de 20 à 98 %, ou allant de 30 à 90 % , ou bien encore allant de 40 à 80 %

Dans un autre exemple de réalisation, la ou les encres colorantes ne contiennent pas de solvant liquide, notamment lorsqu'on utilise une imprimante laser.

Dans un exemple de réalisation, le substrat du dispositif selon l'invention comporte au moins une zone translucide ou transparente. La zone translucide ou transparente peut se superposer en tout ou partie avec les couches d'encre colorante. Il peut être utile qu'une zone du substrat, transparente, soit non revêtue d'encre cosmétique, car cela permet d'appliquer le substrat sur la peau et de comparer la couleur de l'encre à celle de la peau.

L'intégralité des couches d'encres colorantes peut se superposer à la zone translucide ou transparente. En variante, une partie seulement des couches d'encres colorantes se superpose à la zone transparente.

Le substrat peut être réalisé entièrement en un matériau transparent ou translucide. Dans ce cas, la zone translucide ou transparente s'étend sur toute la surface du substrat. En variante, le substrat est opaque sur tout ou partie de sa surface.

Le substrat peut être une plaque rigide. Il peut être en plastique (par exemple polyéthylène ou polystyrène). Il peut être en matière organique ou minérale.

Dans un exemple de réalisation, le substrat comporte un ou plusieurs logements dans lesquels la ou les couches d'encre colorante sont imprimées, ce qui permet d'éviter le mélange entre deux zones de couleurs différentes, notamment dans le cas d'une encre très fluide ou d'une encre sous forme de poudre.

Dans un exemple de réalisation, plusieurs encres colorantes cosmétiques différentes sont présentes au sein d'une même couche d'encre. En variante, tout ou partie des régions portent une couche formée d'une unique encre colorante cosmétique.

Les encres colorantes ont avantageusement été déposées sur le substrat par impression par au moins une imprimante numérique.

Tout ou partie des couches d'encre colorante obtenues par impression peuvent être imprimées sous la forme de points de trame et/ou de lignes de trame, de façon à former une image en demi-ton par exemple, monochromatique ou polychromatique.

La couche d'encre colorante peut comporter plusieurs encres colorantes de couleurs différentes, disposées chacune selon des points de trame.

Tout ou partie des encres colorantes peut être colorée sous observation en lumière blanche dans le domaine visible (400 nm - 800 nm). En variante, les encres colorantes sont incolores en lumière blanche dans le domaine visible mais peuvent apparaître comme colorées après soumission à un stimulus chimique et/ou énergétique, comme l'exposition aux UV (365 nm - 400 nm), par exemple lorsque l'encre colorante contient un pigment photochrome ou fluorescent.

Lorsque la surface d'impression comporte plusieurs régions, les encres colorantes de chaque région diffèrent entre elles, de préférence, au moins de par la couleur qu'elles produisent après application sur les matières kératiniques.

On peut utiliser divers types d'encres colorantes destinées à être appliquées sur différentes zones des matières kératiniques.

Tout ou partie des encres colorantes peuvent être des compositions de maquillage de la peau, par exemple des compositions destinées à être appliquées sur les joues ou les paupières. Tout ou partie des encres colorantes peuvent ainsi être des compositions de fond de teint ou d'eye-liner ou de fard à paupières.

Tout ou partie des encres colorantes peuvent être des compositions de maquillage des lèvres, par exemple des rouges à lèvres ou des gloss.

Tout ou partie des encres colorantes peuvent être des compositions de maquillage des cils, par exemple des compositions de mascara, ou de maquillage des cheveux, ou de masquage des poils.

Les encres colorantes présentes sur un même dispositif peuvent toutes être destinées à être appliquées sur une même zone des matières kératiniques, par exemple sur le visage. Les encres colorantes présentes sur un même dispositif sont par exemple toutes des compositions de fond de teint. En variante, un même dispositif peut comporter deux couches d'encre colorante qui sont chacune destinées à être appliquées sur une zone différente des matières kératiniques. Par exemple, une encre colorante d'une première couche est destinée à être appliquée sur la peau et une encre colorante d'une deuxième couche, différente de l'encre de la première couche, est destinée à être appliquée sur les lèvres ou les cils.

De préférence, tout ou partie des encres colorantes sont aptes à transférer sur les matières kératiniques sans ajout d'un composé fluide, notamment d'un liquide, tiers. En d'autres termes, tout ou partie des encres colorantes peuvent transférer sur les matières kératiniques par simple mise en contact de la zone destinée à être maquillée avec les encres colorantes et ce sans nécessiter l'application d'un liquide tiers destiné à améliorer le transfert des encres, comme dans le cas des décalcomanies.

Dans un exemple de réalisation, au moins l'une des couches d'encre colorante est imprimée sur la surface d'impression du substrat de manière à former un motif reproduisant un aspect d'hétérogénéités de relief et/ou de couleur de la peau.

Dans l'invention, dispositif comporte au moins trois, de préférence au moins quatre, régions, sur chacune desquelles une couche d'encre colorante a été déposée par impression.

Le dispositif peut ou non présenter un ou plusieurs reliefs, par exemple telles que des nervures, séparant tout ou partie des régions portant les encres colorantes. En variante, le dispositif ne comporte pas de relief séparant les régions portant les encres colorantes.

Dans un exemple de réalisation, au moins deux régions portant les couches d'encre colorante sont en contact. En particulier, chacune des régions portant une couche d'encre colorante est en contact avec une ou plusieurs autres régions. En variante, les régions portant les couches d'encre colorante ne sont pas en contact les unes avec les autres.

Le dispositif de maquillage est, de préférence, sous la forme d'une palette. Le dispositif de maquillage peut présenter un rapport longueur/épaisseur supérieur ou égal à 10.

La longueur correspond à la plus grande dimension du dispositif mesurée lorsque celui-ci est observé de face du côté de la surface portant les encres colorantes. L'épaisseur correspond à la plus grande dimension du dispositif lorsque ce dernier est observé de côté.

Dans un exemple de réalisation, les couches d'encres colorantes de plusieurs régions produisent un dégradé de l'effet optique le long d'un chemin reliant ces régions et/ou chacune des régions est associée à un indicateur, de préférence porté par le substrat, permettant de fournir une information sur la localisation de la zone des matières kératiniques sur laquelle la ou les encres colorantes portées par cette région sont destinées à être appliquées.

Au moins une caractéristique colorimétrique choisie parmi L, C*, h, a et b peut évoluer de manière continue entre les différentes régions.

Les différentes composantes de la couleur sont définies dans l'espace colorimétrique CIE1976 (L*, a*, b*) ou CIELAB. La valeur a* correspond à la position sur l'axe rouge/vert et la valeur b* à la position sur l'axe bleu/jaune. La saturation C* correspond à la quantité (a^{∗2} + b^{∗2})^{1/2}. L'angle de teinte h correspond à la quantité arctan (a*/b*). L* désigne la clarté.

Avantageusement, les couches d'encre colorante produisent un dégradé de couleur le long d'un chemin reliant les régions.

L'utilisation d'un dispositif présentant un dégradé de couleur est avantageuse dans la mesure où elle permet à un utilisateur de choisir la couleur qui convient le mieux au teint des matières kératiniques destinées à être maquillées.

Par « dégradé de l'effet optique le long d'un chemin », il faut comprendre que les encres colorantes présentent le long de ce chemin au moins deux zones présentant un degré de l'effet optique distinct, la variation du degré de l'effet optique étant continue ou continue par morceaux entre ces deux régions lorsque l'on se déplace le long dudit chemin.

Le chemin peut être linéaire. En d'autres termes, les encres colorantes peuvent présenter un dégradé de l'effet optique le long d'une direction. En variante, le chemin peut être autre que linéaire, par exemple sous forme de ligne brisée ou curviligne, voire sinusoïdale.

La présence d'indicateurs fournissant une information sur la zone des matières kératiniques à maquiller constitue avantageusement un outil permettant d'aider l'utilisateur à réaliser le maquillage.

### Matière colorante

L'encre colorante peut comporter une ou plusieurs matières colorantes choisies parmi les colorants hydrosolubles, les colorants liposolubles, les matières colorantes pulvérulentes comme les pigments, notamment les nacres, et les paillettes, ou bien encore les polymères colorants.

La ou les matières colorantes peuvent être présentes, dans l'encre colorante, en une teneur allant de 0,01 à 40% en poids, par rapport au poids total de l'encre colorante, de préférence de 0,1 à 30% en poids, préférentiellement allant de 0,5 à 20 % en poids.

Par « pigments », il faut comprendre des particules de toute forme, blanches ou colorées, minérales ou organiques, insolubles dans le milieu cosmétique, destinées à colorer l'encre colorante.

Par « nacres », il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, FD & C et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique ainsi que les pigments nacrés à base d'oxychlorure de bismuth.

Parmi les colorants hydrosolubles, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène. Parmi les colorants liposolubles, on peut citer le Rouge Soudan III (CTFA : D&C Red 17), la lutéine, le vert de quinizarine (CTFA : D&C green 6), le pourpre d'alizurol SS (CTFA : D&C violet n° 2), le brun Soudan, le DC Yellow 11, le DC orange 5, le jaune quinoléine,la curcumine, les dérivés des caroténoïdes tels que le lycopène, le bétacarotène, la bixine ou la capsantéine, et leurs mélanges.Les polymères colorants sont généralement des copolymères à base d'au moins deux monomères distincts dont l'un au moins est un colorant organique monomérique. De tels colorants polymériques sont connus de l'homme du métier. On peut, par exemple, se référer aux documents : US-5,032,670 ; US-4,999,418 ; US-5,106,942; US-5,030,708 ; US-5,102,980; US-5,043,376 ; US-5,104,913; US-5,281,659, US-5,194,463 ; US-4,804,719 ; WO92/07913, ou bien encore EP1048282.

L'encre colorante peut comporter une ou plusieurs matières colorantes, notamment pigments, photochromes, c'est à dire des matières colorantes qui possèdent la propriété de changer de couleur lorsqu'elles sont irradiées par une source lumineuse d'une certaine fréquence, puis de reprendre leur couleur initiale, ou une couleur proche, lorsque l'on arrête l'irradiation. On peut notamment citer parmi les matières colorantes photochromes:
- les composés photochromes minéraux, complexes et plus particulièrement les aluminosilicates dopés et les oxydes et hydrates d'oxydes métalliques, tels que ceux décrits dans WO-A-02/36083,
- les composés photochromes de naphtopyranes, notamment de 3H-naphto-[2,1-b]-pyranes ou de 2H-naphto-[1,2-b]-pyranes comme par exemple le 3,3-di(4-méthoxyphényl)-6-morpholino-3H-naphto[2,1-b] pyrane, le 3-phényl-3-(4-morpholinophényl)-6-morpholino-3H-naphto[2,1-b]pyrane, le 3-phényl-3-(4-pipéridinophényl)-6-morpholino-3H-naphto[2,1-b]pyrane, le 3-phényl-3-(4-pipéridinophényl)-6-carboxyméthyl-9-N-diméthyl-3H-naphto[2,1-b]pyrane , le 2-phényl-2-(4-piperidinophényl)-5-carboxyméthyl-9-N-diméthyl-2H-naphto[1,2-b]pyrane. De tels composés sont décrits dans la demande EP-A-1410785,
- les composés de diaryléthènes ou de fulgides tels que ceux décrits dans la demande EP-A-938887.

L'encre colorante peut comporter en outre une ou plusieurs charges, notamment en une teneur allant de 0,01 % à 50 % en poids, par rapport au poids total de l'encre colorante, de préférence allant de 0,01 % à 30 % en poids.

Par « charges », il faut comprendre des particules de toute forme, incolores ou blanches, minérales ou de synthèse, insolubles dans le milieu de l'encre colorante quelle que soit la température à laquelle cette encre est fabriquée.

Ces charges servent notamment à modifier la rhéologie ou la texture de l'encre colorante.

Les charges peuvent être minérales ou organiques de toute forme, plaquettaires, sphériques ou oblongues, quelle que soit la forme cristallographique (par exemple feuillet, cubique, hexagonale, orthorhombique, etc.). On peut citer le talc, le mica, la silice, le kaolin, les poudres de polyamide (Nylon^{®}) (Orgasol^{®} de chez Atochem), de poly-β-alanine et de polyéthylène, les poudres de polymères de tétrafluoroéthylène (Téflon^{®}), la lauroyl-lysine, l'amidon, le nitrure de bore, les microsphères creuses polymériques telles que celles de chlorure de polyvinylidène/acrylonitrile comme l'Expancel^{®} (Nobel Industrie), de copolymères d'acide acrylique (Polytrap^{®} de la société Dow Corning) et les microbilles de résine de silicone (Tospearls^{®} de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads^{®} de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

L'encre colorante peut comporter en outre un polymère additionnel tel qu'un polymère filmogène. On entend par « polymère filmogène », un polymère apte à former à lui seul ou en présence d'un agent auxiliaire de filmification, un film continu et adhérent sur un support, notamment sur les matières kératiniques. Parmi les polymères filmogènes susceptibles d'être utilisés dans l'encre colorante, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

Bien entendu, l'homme du métier veillera à choisir ce ou ces éventuels composés complémentaires, et/ou leur quantité, de manière telle que les propriétés avantageuses de l'encre colorante ne soient pas, ou substantiellement pas, altérées par l'adjonction envisagée.

### Milieu cosmétiquement acceptable

L'encre colorante selon l'invention constitue un milieu cosmétiquement acceptable, c'est-à-dire compatible avec les matières kératiniques telles que la peau du visage ou du corps, les lèvres, les cheveux, les cils, les sourcils et les ongles.

Dans un exemple de réalisation, tout ou partie des encres colorantes comportent, en outre, un composé de transfert ayant une température d'ébullition supérieure ou égale à 120°C, en particulier allant de 120 °C à 350 °C, notamment allant de 120 °C à 300 °C.

Dans un autre exemple de réalisation, tout ou partie des encres colorantes comportent, en outre, un composé de transfert ayant une température d'ébullition supérieure ou égale à 200°C, en particulier allant de 200 °C à 350 °C.

L'encre peut contenir en outre de l'eau.

Le composé de transfert est avantageusement sous forme liquide à la température ambiante (25 °C).

Le(s) composé(s) de transfert est(sont) de préférence, choisi(s) parmi : le glycérol (température d'ébullition : 290°C), l'éthylène glycol (température d'ébullition : 197°C), le diéthylène glycol (température d'ébullition : 245°C), le triéthylène glycol (température d'ébullition : 285°C), le 1,5 pentane diol (température d'ébullition : 242°C), le 1-pentanol (température d'ébullition : 138 °C), le 1-hexanol (température d'ébullition : 157 °C), l'alcool benzylique (température d'ébullition : 205°C), le 1-hexanal (température d'ébullition : 130 °C), le 1-heptanal (température d'ébullition : 153 °C), le 2 pyrrolidone (température d'ébullition : 245°C), le N-méthyl 2-pyrrolidone (température d'ébullition : 203°C), le N-éthylpyrrolidine (température d'ébullition : 211°C), le carbonate de propylène (température d'ébullition : 240°C), le 1,3 diaminopropane (température d'ébullition : 140°C), le 2-imidazolidinone (température d'ébullition : 131 °C) , le 2-amino-1-butanol (Eb=178 °C), le 2-aminopropanol (température d'ébullition : 173 °C) ,l'ethanolamine (température d'ébullition : 171 °C) ; l'acétate de butyle (température d'ébullition : 126 °C) et leurs mélanges.

Selon un premier mode de réalisation de l'invention, le(s) composé(s) de transfert sont miscible(s) dans l'eau à 25 °C (notamment ayant une solubilité dans l'eau d'au moins 5 % en poids), et sont, de préférence, choisi(s) parmi les mono alcools en C5-C6, les polyols en C2-C6, les esters en C6-C10, les cétones en C5-C8 (notamment cycliques), les aldéhydes en C6-C7, les carbonates cycliques en C3-C8, les urées cycliques en C3-C8, les aminoalcools en C2-C6 , les diamines en C3-C6, les silicones aminées miscibles à l'eau comme la SILICONE QUATERNIUM-8 (nom INCI) par exemple vendue sous la dénomination « SILSENSE Q-Plus Silicone» par NOVEON, le PEG-7 AMODIMETHICONE (nom INCI) par exemple vendue sous la dénomination « SILSENSE A-21 SILICONE » par NOVEON et leurs mélanges.

L'encre colorante peut comporter une pluralité de composés de transfert différents, de préférence au moins trois composés de transfert différents, de préférence au moins quatre composés de transfert différents, les composés de transfert ayant chacun une température d'ébullition supérieure ou égale à 120°C, notamment allant de 120 à 350 °C.

Dans un exemple de réalisation, les composés de transfert comportent un mélange d'au moins deux polyols différents en C2-C6, notamment d'au moins trois polyols différents en C2-C6, notamment d'au moins quatre polyols différents en C2-C6.

Selon un deuxième mode de réalisation de l'invention, le(s) composé(s) de transfert sont non miscible(s) dans l'eau (solubilité dans l'eau à 25 °C inférieure à 5 % en poids). De tels composés de transfert peuvent être choisis parmi les huiles habituellement utilisées en cosmétique, volatiles ou non, qui peuvent être choisies parmi les huiles naturelles ou synthétiques, carbonées, hydrocarbonées, fluorées, éventuellement ramifiées, seules ou en mélange.

On entend par "huile non volatile", une huile susceptible de rester sur la peau à température ambiante et pression atmosphérique au moins une heure et ayant notamment une pression de vapeur à température ambiante (25°C) et pression atmosphérique, non nulle, inférieure à 0,01 mm de Hg (1,33 Pa).

On peut en particulier citer les huiles non volatile carbonées, notamment hydrocarbonées, d'origine végétale, minérale, animale ou synthétique, telles que l'huile de paraffine (ou vaseline), le squalane, le polyisobutène hydrogéné (Parléam), le perhydrosqualène, de macadamia, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales, de beurre de karité; les esters linéaires, ramifiés ou cycliques, ayant plus de 6 atomes de carbone, notamment 6 à 30 atomes de carbone, tels que les esters d'acide lanolique, d'acide oléique, d'acide laurique, d'acide stéarique; les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, en particulier les esters en C12-C36, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de diglycérine; les acides gras supérieurs, notamment en C14-C22, tels que l'acide myristique, l'acide palmitique, l'acide stéarique, l'acide béhénique, l'acide oléique, l'acide linoléique, l'acide linolénique ou l'acide isostéarique; les alcools gras supérieurs, notamment en C16-C22, tels que le cétanol, l'alcool oléique, l'alcool linoléique ou linolénique, l'alcool isostéarique ou l'octyl dodécanol; et leurs mélanges.

On peut encore citer le décanol, le dodécanol, l'octadécanol, les triglycérides liquides d'acides gras de 4 à 10 atomes de carbone comme les triglycérides des acides heptanoïque ou octanoïque, les triglycérides des acides caprylique/caprique; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol; des alcools gras ayant de 12 à 26 atomes de carbone comme l'octyldodécanol, le 2-butyloctanol, le 2-hexyldécanol, le 2-undécylpentadécanol.

Parmi les composés volatils, on peut citer les huiles volatiles non siliconées, notamment les isoparaffines en C8-C16 comme l'isododécane, l'isodécane, l'isohexadécane.

Plus préférentiellement, on peut citer les alcanes liquides à température ambiante, volatils ou non, et plus particulièrement le décane, l'heptane, le dodécane, l'isododécane, l'isohexadécane, le cyclohexane, l'isodécane, et leurs mélanges.

Parmi le(s) composé(s) de transfert non miscible(s) dans l'eau, on peut utiliser l'isododécane (température d'ébullition : 180°C), le myristate d'isopropyle (température d'ébullition : 168°C), l'alcool isostéarylique (température d'ébullition : 331 °C), le néopentanoate d'isodécyle (température d'ébullition : 272 °C), l'isononanoate d'isononyle (température d'ébullition : 285 °C), l'alcool oleylique (température d'ébullition : 315 °C), l'octyl-2 dodécanol (température d'ébullition : 358 °C), le palmitate d'isopropyle (température d'ébullition : 340 °C), l'isostéarate d'isopropyle (température d'ébullition : 361 °C), et leurs mélanges..

L'encre colorante selon l'invention peut également comprendre des cires.

Par « cire », on entend un composé lipophile, solide à température ambiante (25 °C), à changement d'état solide/liquide réversible, ayant un point de fusion supérieur ou égal à 30 °C pouvant aller jusqu'à 120 °C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.

Les cires peuvent être hydrocarbonées, fluorées et/ou siliconées et être d'origine végétale, minérale, animale et/ou synthétique. En particulier, les cires présentent une température de fusion supérieure à 25°C et mieux supérieure à 45°C. Comme cire utilisable dans l'encre colorante, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

La nature et la quantité des cires sont fonction des propriétés mécaniques et des textures recherchées. A titre indicatif, l'encre colorante peut contenir de 0,01 à 30% en poids de cires, par rapport au poids total de l'encre colorante et mieux de 1 à 20 % en poids.

L'encre colorante comporte avantageusement un mélange d'une pluralité de composés de transfert, chacun étant miscible à l'eau à 25 °C. En variante, l'encre colorante comporte un mélange d'une pluralité de composés de transfert, chacun étant non miscible à l'eau. En variante encore, l'encre colorante comporte un mélange d'une pluralité de composés de transfert et comporte, à la fois, un ou plusieurs composé(s) de transfert miscible(s) à l'eau et un ou plusieurs composé(s) de transfert non miscible(s) à l'eau.

Dans un exemple de réalisation, l'encre colorante comporte, en outre, un solvant organique liquide (à 25 °C) ayant une température d'ébullition inférieure à 120°C. Comme exemples de tels solvants organiques , on peut citer, le n-hexane (température d'ébullition : 69 °C), le cyclohexane (température d'ébullition : 81 °C), l'acétate d'éthyle (température d'ébullition : 76 °C), le n-heptane (température d'ébullition : 98 °C) l'acétate d'isobutyle (température d'ébullition : 116 °C), l'acétate de méthyle (température d'ébullition : 57 °C), l'éthanol (température d'ébullition : 78 °C), le butanol (température d'ébullition: 117 °C), l'isopropanol (température d'ébullition : 81 °C), le n-propanol (température d'ébullition : 97 °C), et leurs mélanges.

Sauf mention contraire, toutes les températures d'ébullition sont mesurées à la pression atmosphérique, i.e. 1 013,25 mbar (correspondant à une pression de 1 013,25 hectopascal (hPa)).

L'encre colorante peut se présenter sous la forme d'une solution aqueuse, d'un gel aqueux ou d'une émulsion.

Dans un exemple de réalisation, l'encre colorante comprend une cire et on ramollit par exemple l'encre au moment de l'utilisation par mise à proximité avec un organe de chauffage.

La cire est par exemple portée à une température comprise entre 30°C et 60°C avant son application sur les matières kératiniques. En variante, la cire est portée à une température comprise entre 30°C et 60°C alors qu'elle est en contact avec la zone des matières kératiniques destinée à être maquillée.

Dans le cas où une encre colorante est destinée à être portée à une température comprise entre 30°C et 60°C préalablement à son application, l'encre colorante portée à cette température peut être appliquée sur les ongles afin de réaliser un maquillage de ces derniers.

L'encre colorante peut être portée à une température comprise entre 30°C et 60°C par mise à proximité d'un organe chauffant. En variante, l'encre colorante obtenue juste après l'impression peut déjà être à une telle température, l'utilisateur peut alors appliquer sur les matières kératiniques l'encre colorante à cette température, avant qu'elle ne refroidisse.

Dans un exemple de réalisation, le substrat est en un matériau non absorbant, par exemple en matière plastique. Le substrat est avantageusement non poreux, au moins sur la face destinée à recevoir les encres colorantes.

Le substrat peut ou non être plan.

Dans un exemple de réalisation, une ou plusieurs des régions portant une ou plusieurs encres colorantes sont détachables d'une partie du substrat.

Dans un exemple de réalisation, le substrat comporte une information sur la nature des matières kératiniques destinées à être maquillées par tout ou partie des encres colorantes. Cette information peut être imprimée avec la même encre ou non que l'une de celles présentes sur le substrat.

Dans un exemple de réalisation, lorsqu'une ou plusieurs des encres colorantes sont destinées à être appliquées par transfert sur les joues et/ou les ongles, le substrat peut avoir une épaisseur supérieure ou égale à 1 mm, notamment à 3 mm, notamment allant de 1 à 5 mm.

Dans un exemple de réalisation, lorsqu'une ou plusieurs des encres colorantes sont destinées à être appliquées par transfert sur la région péri-oculaire et/ou sur les lèvres, le substrat peut avoir une épaisseur supérieure ou égale à 3 mm, notamment à 1 cm, notamment allant de 3 à 20 mm.

Dans un exemple de réalisation, lorsqu'une ou plusieurs des encres colorantes sont destinées à être appliquées par transfert sur le nez et/ou dans la région des oreilles, le substrat peut avoir une épaisseur supérieure ou égale à 1 cm, notamment à 3 cm, notamment allant de 1 à 4 cm.

L'épaisseur du substrat précité correspond à sa dimension maximale mesurée perpendiculairement à la surface portant les encres colorantes destinées à être appliquées par transfert sur les matières kératiniques.

Ainsi, le substrat a avantageusement une épaisseur adaptée à la zone des matières kératiniques à maquiller.

Selon un autre de ses aspects, la présente invention concerne un procédé de préparation d'un dispositif tel que défini plus haut, comportant l'étape consistant à déposer à l'aide d'au moins une imprimante sur une région ou une pluralité de régions d'un substrat une encre ou une pluralité d'encres colorantes cosmétiques différentes.

L'imprimante peut être une imprimante jet d'encre (« Inkjet »), par exemple thermique ou piezo-électrique, une imprimante par sublimation ou une imprimante 3D, ou une imprimante laser, notamment à four désactivé. L'imprimante peut être une imprimante jet d'encre alimentaire de type Gatocopy A426 permettant l'impression sur des objets non plans.

Dans un exemple, l'imprimante est une imprimante laser agencée pour permettre la formation par électrophotographie ou magnétophotographie d'une couche d'encre présentant un motif sur une surface de transfert à partir d'au moins un toner cosmétique et pour délivrer le toner présent sur la surface de transfert dans un état suffisamment libre pour permettre son prélèvement ou son transfert par contact avec les matières kératiniques humaines.

Par "toner cosmétique", il faut comprendre une composition cosmétique pulvérulente compatible avec la formation d'une image par un procédé électrophotographique ou de magnétophotographique tels que mis en oeuvre au sein des imprimantes laser. De préférence, il s'agit d'un toner qui convient à une mise en oeuvre par voie électrophotographique.

Le toner est cosmétique en ce sens qu'il est compatible avec une application sur les matières kératiniques humaines. Selon la surface à maquiller, la formulation du toner pourra être différente. Par exemple, pour une application sur les cheveux ou les ongles, il est possible d'utiliser certains composés qui ne pourraient pas être utilisés pour une application sur les lèvres, par exemple.

Lorsque l'encre est sous forme de toner cosmétique, ce toner peut comporter outre un agent de coloration, un composé de contrôle de la charge électrique, une charge additionnelle particulière, un lubrifiant, une cire et/ou un liant.

De préférence, les particules du toner ont une taille moyenne comprise entre 1 et 16 µm. Le toner comporte par exemple des pigments présentant une taille de particules comprise entre 1 et 10µm.

Avantageusement, l'impression met en oeuvre plusieurs encres de couleurs différentes.

L'impression peut mettre en oeuvre au moins trois, notamment au moins quatre, cinq, six, sept, huit, neuf, dix, onze ou douze, encres colorantes de couleurs différentes.

L'impression peut mettre en oeuvre uniquement des encres colorantes produisant des couleurs primaires. En variante, l'impression met en oeuvre à la fois des encres colorantes correspondant à des couleurs primaires et au moins une encre colorante correspondant à une couleur non primaire.

L'impression des encres colorantes peut être une impression en trichromie ou en quadrichromie.

Dans un exemple de réalisation, le procédé comporte une étape de choix d'un ensemble d'encres colorantes à imprimer par un utilisateur et de transmission, par un appareil relié à au moins une imprimante réalisant l'impression, d'une information associée à cet ensemble.

L'appareil peut être un ordinateur, un téléphone mobile évolué, encore appelé "smartphone", ou une tablette tactile. L'appareil peut être relié physiquement et/ou par le biais d'un réseau d'échange de données à ladite imprimante.

Dans un exemple de réalisation, le procédé comporte une étape de récupération d'une information relative à la nature de la couche d'encre colorante à imprimer stockée sur un support informatique, l'impression étant effectuée en fonction de cette information.

Par exemple, le procédé comporte une étape de choix par un utilisateur d'un ensemble d'encres colorantes à imprimer parmi plusieurs ensembles d'encres colorantes proposés à l'utilisateur, des informations relatives aux ensembles proposés étant stockées sur un support informatique, le procédé comportant en outre une étape de transmission, par un appareil relié à au moins une imprimante réalisant l'impression, d'une information associée à l'ensemble choisi.

L'appareil peut être relié physiquement et/ou par le biais d'un réseau d'échange de données au support informatique.

L'invention offre avantageusement la possibilité d'échanger entre utilisateurs des fichiers relatifs aux dispositifs de maquillage à imprimer.

Ainsi, l'utilisateur peut choisir une encre colorante ou un ensemble d'encres colorantes à imprimer parmi plusieurs ensembles d'encres colorantes proposés, ces ensembles ayant étant créés par des artistes et/ou par d'autres utilisateurs.

Dans un exemple de réalisation, un ou plusieurs ensembles d'encres colorantes à imprimer ainsi qu'un conseil de maquillage associé à ces ensembles peuvent être proposés à l'utilisateur. Le conseil de maquillage peut fournir une information sur la manière d'appliquer les encres colorantes sur les matières kératiniques et/ou sur la zone des matières kératiniques à maquiller et/ou peut proposer une composition de maquillage complémentaire destinée à être appliquée en combinaison avec tout ou partie des encres colorantes à imprimer.

Dans un exemple de réalisation, l'utilisateur reçoit un conseil personnalisé sur le dispositif de maquillage à fabriquer. Par exemple, l'utilisateur reçoit un fichier informatique fournissant une information sur l'effet optique produit par les encres colorantes des couches à imprimer et/ou sur la nature de ces dernières et/ou sur la zone des matières kératiniques sur lesquelles les encres colorantes des couches sont destinées à être appliquées. Le fichier informatique peut être transmis à l'utilisateur par le biais d'un réseau d'échange de données, par exemple par Internet.

En variante, l'utilisateur conçoit le dispositif de maquillage à réaliser et transmet pour fabrication du dispositif un fichier informatique fournissant une information sur l'effet optique produit après application sur les matières kératiniques par les encres colorantes des couches à imprimer et/ou la nature de ces dernières et/ou la zone des matières kératiniques sur lesquelles les encres colorantes sont destinées à être appliquées. Le dispositif peut alors être réalisé automatiquement à partir du fichier transmis.

Dans un exemple de réalisation, le procédé comporte, avant l'impression, une étape de simulation de l'aspect des matières kératiniques revêtues d'une ou plusieurs des encres colorantes à imprimer, la simulation de l'aspect des matières kératiniques revêtues étant affichée sur un écran d'un appareil.

Avantageusement, une telle simulation permet à l'utilisateur de se voir à l'écran dans des configurations de maquillage, l'aidant ainsi à optimiser son dispositif de maquillage à imprimer.

Dans un exemple de réalisation, le choix des encres colorantes est réalisé en fonction d'une information relative à l'aspect des matières kératiniques à maquiller, notamment après réalisation d'une acquisition d'au moins une image des matières kératiniques à maquiller et/ou réalisation d'au moins une mesure d'au moins une caractéristique optique de ces matières kératiniques.

Dans un exemple de réalisation, le procédé comporte une étape d'enregistrement, sur un support de stockage de données, d'une information relative au dispositif préparé, l'information étant de préférence relative à la nature des encres colorantes présentes sur le dispositif préparé et/ou à la nature des matières kératiniques destinées à être revêtues par tout ou partie des encres colorantes du dispositif préparé.

Ainsi, l'invention permet avantageusement à l'utilisateur de garder en mémoire son dispositif de maquillage idéal. Le fait d'utiliser une imprimante lui assure alors une reproductibilité parfaite à chaque nouvelle impression.

Dans un exemple de réalisation, ladite au moins une imprimante effectue un premier passage d'impression permettant d'obtenir une première fraction du dépôt des encres colorantes puis au moins un deuxième passage d'impression permettant d'obtenir une deuxième fraction du dépôt des encres colorantes superposée à la première fraction. Ainsi, pour imprimer la ou les couches on peut déposer les encres colorantes en plusieurs passes d'impression.

Le fait de réaliser plusieurs passes d'impression peut permettre, en augmentant la quantité d'encres colorantes déposées sur le substrat, d'améliorer la durée d'utilisation des dispositifs selon l'invention.

Dans un exemple de réalisation, tout ou partie des encres colorantes imprimées forment un motif transférable sur les matières kératiniques. Le motif peut être transféré par pression de la surface d'impression du substrat sur les matières kératiniques, au doigt ou avec un applicateur tel qu'un rouleau, une brosse, un pinceau, ou une éponge, notamment réalisée en mousse synthétique.

Le motif formé par les encres colorantes peut comporter plusieurs zones de couleurs différentes. En variante, le motif est un aplat de couleur.

En variante, les encres colorantes peuvent former un dégradé de couleur et sont notamment destinées à être appliquées dans la zone péri-oculaire. En variante, tout ou partie des encres colorantes forment un motif reproduisant l'aspect d'hétérogénéités de relief et/ou de couleur de la peau, par exemple un grain de peau ou des taches de rousseur, ou encore des faux sourcils. Dans ce cas, l'application peut se faire par transfert, afin de conserver le motif imprimé.

Le substrat peut être réutilisable. Par exemple, une impression est réalisée sur le substrat, lequel est accessible, mais ne sort pas de l'imprimante. Ainsi, après utilisation, l'imprimante peut réintégrer le substrat, le nettoyer et le rendre prêt pour une nouvelle impression.

Selon un autre de ses aspects, il est aussi présenté un procédé de maquillage des matières kératiniques humaines, comportant l'étape consistant à appliquer tout ou partie d'au moins une encre colorante présente sur un dispositif sur les matières kératiniques humaines, notamment sur la peau, notamment sur la joue et/ou les paupières.

Dans un exemple de réalisation, les matières kératiniques destinées à être revêtues par tout ou partie des encres colorantes n'ont pas été recouvertes, avant l'application, par un composé fluide tiers destiné à améliorer l'application des encres colorantes et/ou le procédé est dépourvu d'une étape d'ajout à tout ou partie des encres colorantes portées par le substrat d'un composé fluide tiers destiné à améliorer l'application.

Ainsi, la zone des matières kératiniques destinée à être maquillée n'a avantageusement pas été prétraitée au moment de l'application.

En variante, la zone des matières kératiniques destinée à être maquillée a été recouverte, avant l'application, avec un composé fluide tiers comme par exemple de l'éthanol ou de l'isododécane permettant d'améliorer l'application de tout ou partie des encres colorantes et/ou un composé fluide tiers destiné à améliorer l'application a été ajouté à tout ou partie des encres colorantes portées par le substrat avant l'application.

Dans un exemple de réalisation, l'ajout du composé fluide tiers permettant d'améliorer l'application ne permet de solubiliser ou de plus fluidifier que tout ou partie des encres colorantes et non par exemple le substrat du dispositif de maquillage et/ou une couche d'adhésif éventuellement présente.

Dans un exemple de réalisation, une pluralité d'encres colorantes sont mélangées avant l'application sur les matières kératiniques et tout ou partie du mélange est appliqué sur les matières kératiniques.

Les encres colorantes peuvent être mélangées sur le substrat par exemple dans une zone du substrat ne portant initialement aucune encre colorante ou en variante être mélangées sur un support non solidaire du substrat.

Dans un exemple de réalisation, tout ou partie des encres colorantes sont appliquées sur une région des matières kératiniques présentant une hétérogénéité de couleur, par exemple une tache ou une dyschromie par exemple présente sur le visage.

Dans un exemple de réalisation, de l'encre colorante prélevée sur une première couche est appliquée sur une première zone des matières kératiniques et de l'encre colorante prélevée sur une deuxième couche, différente de la première, est appliquée sur une deuxième zone des matières kératiniques, différente de la première.

Le maquillage obtenu peut résulter de la juxtaposition de plusieurs encres colorantes sur les matières kératiniques, par exemple sur le visage.

En variante, on peut, dans un premier temps, déposer plusieurs encres colorantes de manière juxtaposée sur les matières kératiniques puis mélanger ces dernières afin d'obtenir le maquillage.

En variante, on peut, dans un premier temps, déposer plusieurs encres colorantes de manière juxtaposée sur les matières kératiniques, par exemple sur la paupière et/ou les joues, puis réaliser un étalement de tout ou partie des dépôts d'encres colorantes réalisés, par exemple afin d'obtenir un effet de dégradé. Avantageusement, les encres colorantes déposées ne se mélangent pas après l'étalement.

Dans un exemple de mise en oeuvre selon l'invention, le procédé comporte, en outre, une étape de finition du maquillage obtenu sur les matières kératiniques, par exemple de manière à estomper les démarcations entre une zone maquillée et une zone non maquillée. La finition du maquillage obtenu peut comporter une étape d'étalement d'une ou plusieurs encres colorantes pour réaliser un estompage, par exemple.

La finition peut s'effectuer en exerçant une friction sur une partie seulement du maquillage réalisé, par exemple sa partie supérieure dans le cas d'un maquillage réalisé sur la paupière.

Lorsque tout ou partie des encres colorantes forment un motif destiné à transférer sur les matières kératiniques, l'utilisateur peut réaliser une finition avant le transfert des encres colorantes sur les matières kératiniques.

Dans un exemple de réalisation, le procédé comporte ainsi une étape de finition du motif formé par les encres colorantes portées par le substrat, la finition s'effectuant par exemple en exerçant une friction sur une partie seulement du motif, par exemple sa partie supérieure dans le cas d'un motif destiné à être appliqué sur la paupière.

Dans un exemple de réalisation, le procédé comporte, en outre, une étape d'application d'une composition cosmétique complémentaire, différente des encres colorantes, ladite composition cosmétique complémentaire étant, de préférence, proposée à l'utilisateur par un appareil en fonction de la nature d'au moins l'une des encres colorantes présentes sur le dispositif et/ou de la nature des matières kératiniques destinées à être revêtues par tout ou partie des encres colorantes du dispositif.

L'utilisation d'une telle composition complémentaire permet avantageusement d'obtenir des maquillages particulièrement esthétiques, et par exemple d'obtenir des effets de bronzage et/ou d'ombrage esthétiques.

Dans un exemple de réalisation, l'encre colorante est prélevée du dispositif à l'aide d'un applicateur et est appliquée sur les matières kératiniques à l'aide dudit applicateur.

L'applicateur peut être choisi parmi : un rouleau applicateur, un tampon applicateur, un élément en feuille, un patch, un masque, une mousse poreuse, une éponge, une lingette, une brosse, un pinceau, une spatule ou un embout floqué.

L'applicateur peut retenir l'encre colorante par capillarité.

En variante, tout ou partie des encres colorantes présentes sur le substrat sont appliquées par transfert sur les matières kératiniques.

Dans un exemple de réalisation, l'application de l'encre colorante sur les matières kératiniques se fait sans mise en contact de l'encre colorante avec un composé fluide tiers, notamment un liquide tiers.

Selon un autre de ses aspects, il est présenté un ensemble cosmétique comportant au sein d'un même conditionnement un dispositif de maquillage tel que défini plus haut et un applicateur pour l'application de tout ou partie des encres colorantes sur les matières kératiniques humaines.

Les dispositifs de maquillage peuvent différer de par la nature des encres qu'ils portent, notamment de par les couleurs de celles-ci.

Selon encore un autre de ses aspects, la présente invention concerne un ensemble cosmétique comportant au sein d'un même conditionnement un dispositif de maquillage tel que défini plus haut et une composition cosmétique, notamment de maquillage, complémentaire destinée à être appliquée en combinaison avec tout ou partie des encres colorantes portées par le dispositif.

### Description des figures

L'invention pourra être mieux comprise à la lecture de la description qui va suivre, d'exemples de mise en oeuvre non limitatifs de celle-ci, et à l'examen du dessin annexé, sur lequel :
- la figure 1 représente un exemple de dispositif de maquillage,
- la figure 2 illustre la mise en oeuvre d'un procédé de maquillage selon l'invention,
- la figure 3 est un schéma en blocs illustrant des étapes d'un procédé de préparation d'un dispositif de maquillage,
- les figures 4 à 8 représentent des variantes de dispositifs de maquillage,
- les figures 9 et 10 illustrent des variantes de procédés de maquillage selon l'invention,
- la figure 11 est un schéma blocs illustrant des détapes d'une variante d'un procédé de préparation d'un dispositif, et
- les figures 12 à 14 représentent des exemples de réalisation d'ensembles cosmétiques.

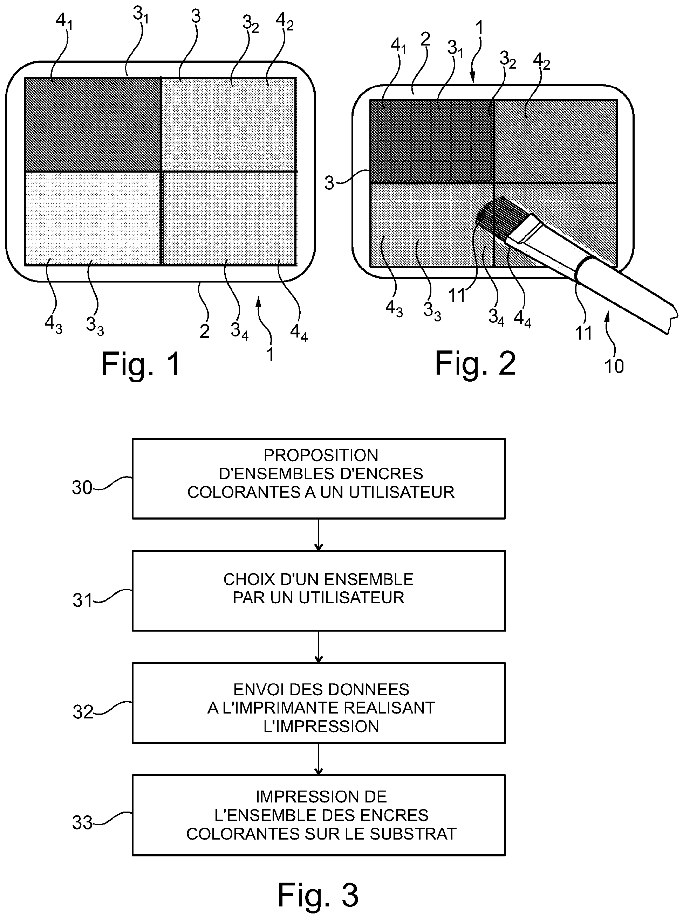

On a représenté à la figure 1 un exemple de dispositif 1 de maquillage. Le dispositif 1 comporte un substrat 2 présentant une surface d'impression 3 divisée en plusieurs régions 3₁, ..., 3₄ comportant chacune une couche d'encre colorante cosmétique différente 4₁, ..., 4₄. Comme illustré, les couches d'encres colorantes 4₁, ..., 4₄ peuvent être de différentes couleurs, par exemple de même teinte et de saturation différente, ou de teintes différentes et même saturation, ou de teintes et saturations différentes.

Le substrat 2 est de préférence en un matériau souple. En variante, le substrat 2 est en un matériau rigide ou semi-rigide.

Tout ou partie de la face du substrat 2 disposée du côté des couches d'encres colorantes 4₁, ..., 4₄ est de préférence lisse et présente une rugosité inférieure ou égale à 1 mm, notamment comprise entre 1 et 100 µm, de préférence inférieure ou égale à 50 µm. La rugosité est mesurée à l'aide d'un rugosimètre dont la pointe présente un rayon de courbure de 10 mm et dont l'effort, appliqué sur le matériau à caractériser, est de 6 mN.

Comme illustré, les régions 3₁, ..., 3₄ sont en contact les unes avec les autres et ne sont pas séparées par des reliefs.

La résolution de l'impression au niveau de tout ou partie des couches d'encres colorantes 4₁, ..., 4₄ peut être comprise entre 16 dpi et 1600 dpi.

Dans l'exemple de la figure 1, chaque encre est déposée sous la forme d'un aplat de couleur, d'étendue par exemple supérieure ou égale à 2 cm².

La couleur d'une couche 4i peut résulter d'une synthèse soustractive de couleurs élémentaires déposées sur la région correspondante, ces couleurs élémentaires étant déposées chacune par impression, sous la forme de points de trame par exemple. En variante, une seule encre est déposée sur une région et la couleur de la couche correspond à la couleur propre de l'encre qui est déposée.

Par exemple, pour créer sur une région une couche d'encre de couleur rouge, on peut appliquer sur le substrat une encre intrinsèquement rouge, ou des encres magenta et jaune sous la forme de points de trame superposés.

Le prélèvement d'encre colorante à partir d'une couche 4i imprimée dans une région 3i du dispositif 1 de la figure 1 peut s'effectuer, comme illustré à la figure 2, à l'aide d'un applicateur 10 comportant une partie de préhension 12 et une partie d'application 11. L'applicateur 10 peut, comme illustré, être sous la forme d'un pinceau.

La partie d'application 11 ainsi chargée d'encre(s) colorante(s) est alors mise en contact avec les matières kératiniques à maquiller. L'utilisateur peut, avant l'application, mélanger à l'aide de la partie d'application 11 plusieurs couches d'encre colorante 4₁, ..., 4₄ présentes sur des régions différentes et appliquer le mélange ainsi obtenu sur les matières kératiniques. Le mélange peut s'effectuer sur la surface d'impression 3 du substrat 2 ou sur un support distinct, voire *in situ* sur la peau.

On va maintenant décrire un exemple de procédé de fabrication d'un dispositif selon l'invention, en se référant à la figure 3.

Dans une première étape 30, différents ensembles d'encres colorantes sont proposés à l'utilisateur, par exemple par affichage sur un écran d'un appareil. L'étape 31 de choix de l'ensemble d'encres colorantes à imprimer par l'utilisateur peut se faire sur un écran tactile par exemple.

L'appareil peut, en outre, être agencé pour fournir à l'utilisateur une simulation du résultat de maquillage. Ainsi, l'appareil peut afficher une simulation de l'aspect des matières kératiniques maquillées avec une ou plusieurs des encres colorantes de l'ensemble choisi. Pour ce faire, l'appareil peut acquérir au moins une image des matières kératiniques à maquiller.

Dans une variante, l'utilisateur réalise un fichier informatique répertoriant l'ensemble des encres colorantes qu'il souhaite imprimer.

Une fois l'ensemble des encres colorantes choisi, l'appareil à l'étape 32 envoie à l'imprimante les données nécessaires à l'impression des encres colorantes sur le substrat.

L'appareil peut être relié physiquement et/ou par le biais d'un réseau à l'imprimante réalisant l'impression.

Une fois les données reçues, les encres colorantes sont imprimées sur le substrat à l'étape 33.

Le pilote de l'imprimante peut comporter un menu permettant de sélectionner les cartouches d'encres cosmétiques à utiliser parmi d'autres cartouches en place dans l'imprimante et/ou la nature du substrat qui est imprimé. En variante, l'imprimante reconnait automatiquement que les cartouches à utiliser comportent des encres cosmétiques et ajuste les paramètres de fonctionnement en conséquence. Les cartouches peuvent ainsi comporter un identifiant, par exemple une puce électronique, permettant de fournir à l'imprimante une information relative à la nature des encres colorantes qu'elles contiennent, notamment que celles-ci sont de nature cosmétique.

Dans un exemple de réalisation, l'imprimante est configurée pour interdire l'impression si la présence d'une cartouche comportant une composition non destinée à être mise en contact avec les matières kératiniques humaines, notamment la peau, les ongles ou les lèvres, est détectée.

En variante, l'imprimante peut réaliser une impression même si la présence d'une cartouche comportant une composition non destinée à être mise en contact avec les matières kératiniques humaines, notamment la peau, les ongles ou les lèvres, est détectée, cette cartouche d'encre non cosmétique pouvant être utilisée pour imprimer sur le substrat une information relative à l'une au moins des encres colorantes cosmétiques portées par le substrat et/ou à la nature des matières kératiniques à maquiller.

L'impression du substrat peut se faire en plusieurs passes, pour effectuer des dépôts d'encre successifs au même emplacement, afin d'accroître la quantité d'encre déposée sur le substrat. Le substrat peut effectuer par exemple entre 1 et 20 passages dans l'imprimante et la quantité en matières sèches d'encre cosmétique déposée va par exemple de 0,01 mg/cm² à 100 mg/cm², voire de 0,1 mg/cm² à 10 mg/cm², mieux de 0,2 mg/cm² à 10 mg/cm², en particulier de 0,2 mg/cm² à 5mg/cm².

L'imprimante peut être agencée pour détecter si l'encre précédemment déposée sur le substrat est suffisamment sèche avant d'effectuer l'impression d'une nouvelle couche d'encre, par exemple par mesure de la conduction électrique entre deux points.L'imprimante et/ou le pilote d'imprimante peuvent être réalisés de façon à informer l'utilisateur de la nécessité d'attendre une durée prédéfinie avant d'effectuer une nouvelle impression sur le substrat déjà imprimé. L'imprimante et/ou le pilote peuvent suspendre automatiquement l'impression d'un substrat déjà imprimé tant qu'une durée suffisante ne s'est pas écoulée pour induire un séchage suffisant. L'imprimante est de préférence agencée pour ne pas délivrer le substrat imprimé tant que toutes les couches d'encre à imprimer ne l'ont pas été.

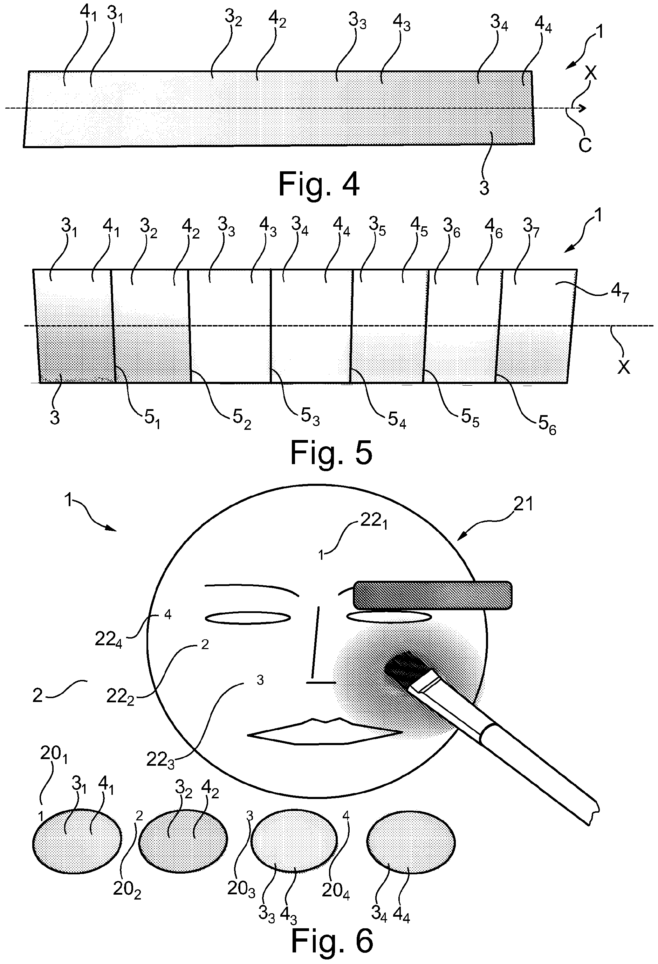

On a représenté à la figure 4 une variante d'un dispositif 1 dans lequel les couches d'encres colorantes 4₁, ..., 4₄ produisent un dégradé de couleur le long d'un chemin C reliant les régions 3₁, ..., 3₄. Le chemin C peut, comme illustré, être linéaire. On ne sort pas du cadre de la présente invention si le chemin a une autre forme.

Comme illustré à la figure 4, les régions 3₁, ..., 3₄ forment une bande qui s'étend selon un axe longitudinal X, ici confondu avec le chemin C.

Au moins une caractéristique colorimétrique choisie parmi L, C*, h, a et b peut évoluer de manière continue entre les différentes régions 3₁, ..., 3₄. Aucune démarcation visible peut n'être présente entre les différentes régions 3₁, ..., 3₄, comme illustré.

On a représenté à la figure 5 une variante de réalisation dans laquelle le dispositif 1 comporte une pluralité de régions se succédant le long d'un axe longitudinal X. Chacune des régions porte une couche d'encre colorante produisant un aplat de couleur différent. Les régions 3₁, ..., 3₇ forment, comme à la figure 4, une bande. En revanche, à la différence de la figure 4, deux régions successives présentent entre elles une démarcation visible 5₁, ..., 5₆.

On a représenté à la figure 6 un exemple de dispositif 1 de maquillage selon l'invention, dans lequel chacune des régions 3₁, ..., 3₄ est associée à un indicateur 20₁, ..., 20₄ porté par le substrat 2, permettant de fournir une information sur la localisation de la zone des matières kératiniques sur laquelle l'encre colorante portée par cette région est destinée à être appliquée. L'application peut se faire au doigt ou avec un applicateur tel qu'un rouleau, une brosse, un pinceau, ou une éponge, notamment réalisée en mousse synthétique.

Le dispositif 1 peut comme illustré comporter une représentation 21 de la zone des matières kératiniques à maquiller, par exemple du visage dans l'exemple illustré. Cette représentation 21 peut faire apparaître la position à maquiller par les différentes encres colorantes par une mention apparente 22₁, ..., 22₄, laquelle peut être identique à l'indicateur 20₁, ..., 20₄.

Les indicateurs 20₁, ..., 20₄ peuvent comporter des symboles alphanumériques et/ou géométriques.

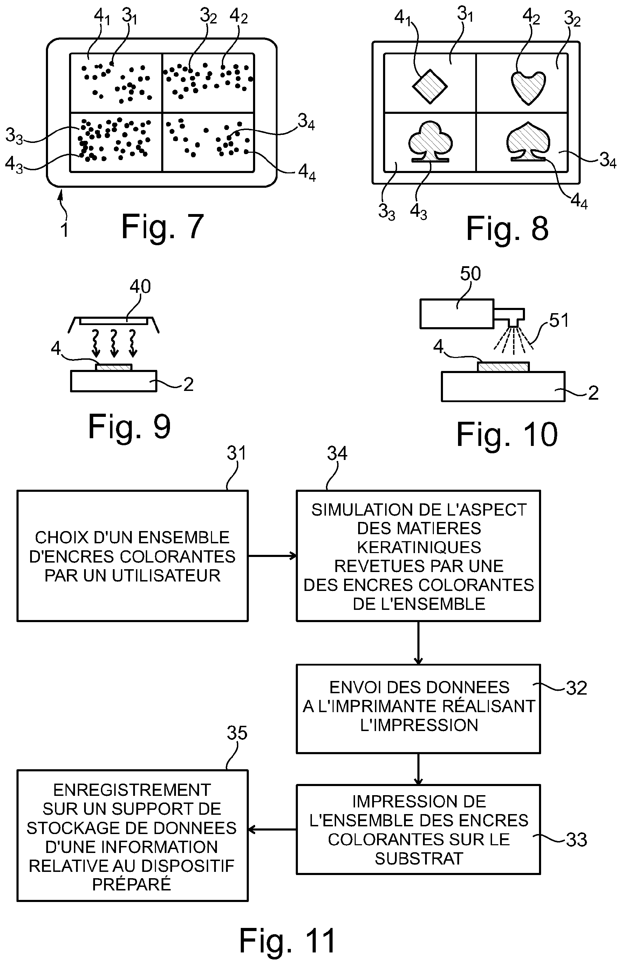

On a représenté à la figure 7 un dispositif dans lequel les différents couches d'encres colorantes 4₁, ..., 4₄ produisent des effets optiques visibles différents d'une couche à l'autre et autres que la couleur, par exemple sont de brillance différente. Les régions 3ᵢ peuvent être de même couleur et différer ainsi uniquement de par leur brillance. En variante, d'une région à l'autre, à la fois la couleur et la brillance varient.

Tout ou partie des encres colorantes peuvent chacune former un motif comme illustré à la figure 8. Les motifs peuvent être monochromes ou polychromes. Dans cet exemple de réalisation, tout ou partie des encres colorantes peuvent être appliquées par transfert. Par exemple, les différentes régions 3₁, ..., 3₄ sont détachables du reste du substrat 2 et sont configurées pour permettre l'application du motif par transfert par pression du substrat sur les matières kératiniques à maquiller.

On a illustré à la figure 9 une variante de procédé, dans laquelle une couche d'encre colorante 4 est exposée à la chaleur d'un organe chauffant 40 de telle sorte que la couche d'encre colorante 4 soit fluidifié ou rendu plus fluide après chauffage.

La couche d'encre colorante ainsi réchauffée est ensuite appliquée sur les matières kératiniques à maquiller, éventuellement après une période de temps suffisante pour que la couche d'encre colorante perde par exemple au moins 5°C, par rapport à la température maximale obtenue du fait du chauffage, mais reste encore suffisamment chaude.

Dans la variante illustrée à la figure 10, un solvant tel que de l'eau 51 est pulvérisé sur la couche d'encre 4. Cette dernière est par exemple sous forme solide, et le solvant ainsi pulvérisé permet de l'humecter. L'encre peut encore ne pas être solide mais le solvant qui est pulvérisé permet de la rendre plus fluide. L'encre colorante 4, une fois humectée, est ensuite appliquée les matières kératiniques. Le solvant est par exemple pulvérisé à l'aide d'un récipient 50 pressurisé de type aérosol, actionné par l'utilisateur.

Le procédé de préparation d'un dispositif peut comporter, comme illustré à la figure 11, une étape 34 de simulation de l'aspect des matières kératiniques revêtues par tout ou partie des encres colorantes de l'ensemble choisi à l'étape 31. Le résultat de cette simulation peut être affiché sur l'écran d'un appareil, par exemple sur le même écran que celui sur lequel les encres colorantes ont été proposées à l'utilisateur, à l'étape 31.

En variante, une simulation de l'aspect des matières kératiniques maquillées est fournie en même temps que l'ensemble d'encres colorantes est proposé à l'utilisateur et avant le choix de ces dernières. A l'issue de l'impression, une information relative à la nature des encres colorantes sélectionnées peut être enregistrée sur un support de stockage de données, par exemple en vue d'une réimpression future.

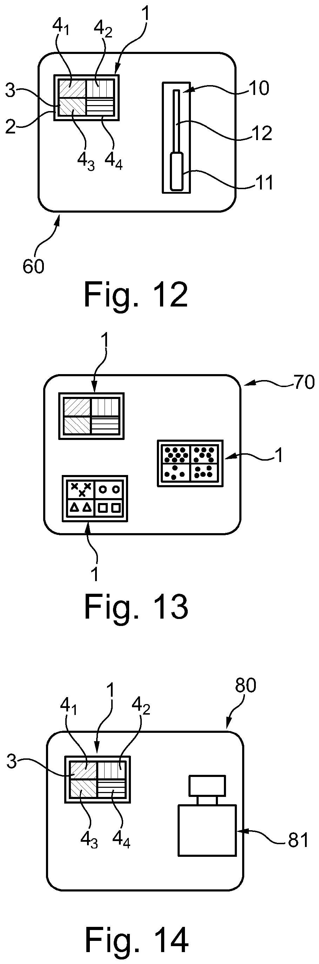

On a représenté à la figure 12 un exemple d'ensemble cosmétique 60. Celui-ci comporte, au sein d'un même conditionnement, un dispositif 1 de maquillage, sous la forme d'une palette portant différentes couches d'encres colorantes 4₁, ..., 4₄, ainsi qu'un applicateur 10 comportant une partie de préhension 12 et une partie d'application 11 destinée à venir prélever tout ou partie des encres colorantes en vue de leur application sur les matières kératiniques.

On a représenté à la figure 13 un autre exemple d'ensemble cosmétique 70. Celui-ci comporte, au sein d'un même conditionnement, une pluralité de dispositifs 1, chacun différant de par la nature des encres colorantes qu'il porte. On peut avoir au sein de ce conditionnement des dispositifs 1 qui diffèrent de par la nature des matières kératiniques destinées à recevoir les encres colorantes qu'il porte. Par exemple, un premier dispositif peut ne comporter que des encres colorantes destinées à être appliquées sur la peau et un second dispositif, différent du premier, peut ne comporter que des encres colorantes destinées à être appliquées sur les lèvres ou les cils.

On a représenté à la figure 14 un autre exemple de réalisation d'un ensemble cosmétique 80. Celui-ci comporte, au sein d'un même conditionnement, un dispositif 1 ainsi qu'une composition cosmétique 81, notamment de maquillage, complémentaire, destinée à être appliquée sur les matières kératiniques en combinaison avec tout ou partie des encres colorantes.

Dans les exemples des figures 12 et 14, les conditionnements peuvent être à fermeture étanche, de façon à éviter le séchage des encres. Les conditionnements peuvent être réalisés avec des moyens permettant d'éviter un contact des encres avec une surface autre que le substrat, de façon à réduire le risque d'un transfert prématuré. Par exemple, le conditionnement comporte une coque thermoformée dont la paroi s'étend à distance des zones du substrat recouvertes d'encres.

### Exemple

Quatre encres colorantes répondant aux formulations fournies dans le tableau ci-dessous ont été réalisées :

**Tableau 1**

| | **Jaune I** | **Magenta I** | **Cyan I** | **Noir I** |
|---|---|---|---|---|
| *Colorant* | D&C Yellow 8 1% | FD&C Red 4 1% | FD&C Blue 1 1% | (1) 1% |
| Ethylène glycol | | 4% | 6% | 5% |
| Diéthylène glycol | 8% | | | |
| 1,5 pentanediol | | 4% | 4% | |
| 2 pyrrolidone | 5% | 5% | 4% | |
| Glycérol | 8% | 3% | 4% | 7% |
| 2 imidazolidinone | 4% | 4% | 4% | 9% |
| Eau | 76% | 79% | 77% | 78% |
| Total | 100% | 100% | 100% | 100% |

| | | | | |
|---|---|---|---|---|
| (1) Brown-Replacement-J de chez Sensient | | | | |

Ces compositions sont introduites dans des cartouches d'imprimante Canon, puis utilisées avec une imprimante à jet d'encre Canon Prixma IP100 configurée pour imprimer une palette de maquillage comportant quatre régions, sous forme d'aplats de couleurs correspondant chacune à une couche d'une seule encre. L'impression est réalisée sur une feuille plastique transparente pour imprimante du commerce (coté lisse) en utilisant chacune des encres indépendamment.

Après chaque impression, on attend huit minutes, puis on prélève, notamment à l'aide d'un pinceau, tout ou partie des encres colorantes déposées pour les appliquer sur une zone de peau, par exemple du bras.

L'expression « comportant un(e) » doit se comprendre comme étant synonyme de « comportant au moins un(e) ».L'expression « compris(e) entre ... et ... » ou « allant de ... à ... » doit se comprendre comme incluant les bornes.

## Revendications

1. Procédé de préparation d'un ensemble cosmétique (60) comportant au sein d'un même conditionnement un dispositif de maquillage (1) comportant un substrat (2) fait en un matériau rigide ou semi-rigide et définissant une surface d'impression (3) présentant au moins trois régions (3₁, ..., 3ₙ) chacune imprimée avec au moins une couche d'encre colorante (4₁, ..., 4ₙ) différente destinée à être appliquée sur les matières kératiniques humaines, dont une couche peut comporter une ou plusieurs encres,
les couches d'encre étant différentes de par la nature chimique de la ou des encres qui les composent et/ou de par les proportions relatives de chaque encre qui les composent, lesdites au moins trois encres colorantes
- ayant été déposées par impression sur la surface d'impression (3) par au moins une imprimante numérique
- n'étant pas recouvertes par un adhésif, et
- produisant après application sur les matières kératiniques au moins un effet optique visible parmi la couleur et/ou la brillance, le dispositif (1) comportant notamment au moins trois, de préférence au moins quatre régions (3₁, ..., 3ₙ) et
le procédé comportant l'étape consistant à imprimer à l'aide d'au moins une imprimante, notamment numérique, au moins une couche d'encre colorante cosmétique (4₁, ..., 4ₙ) sur au moins trois régions (3₁, ..., 3ₙ) d'une surface d'impression (3) d'un substrat (2), notamment imprimer une couche d'encre colorante différente sur chaque région d'une pluralité de régions,
le procédé étant **caractérisé en ce que**
le dispositif comporte un applicateur pour l'application de tout ou partie des encres colorantes du dispositif sur les matières kératiniques humaines, et **en ce que**
l'imprimante est une imprimante à jet d'encre ou une imprimante laser.

2. Procédé selon la revendication 1, ladite au moins une couche (4₁, ..., 4ₙ) comportant une encre ou plusieurs encres destinées à être appliquées sur les matières kératiniques humaines et aptes à produire un maquillage par application sur les matières kératiniques sans ajout d'un composé fluide tiers.

3. Procédé selon l'une quelconque des revendications précédentes, au moins deux couches d'encre colorante (4₁, ..., 4ₙ) produisant un dégradé d'un même effet optique, de préférence la couleur, le long d'un chemin (C) reliant les régions (3₁, ..., 3ₙ) et/ou ladite au moins une région (3₁, ..., 3ₙ) étant associée à un indicateur (20₁, ..., 20ₙ), de préférence porté par le substrat (2), permettant de fournir une information sur la localisation de la zone des matières kératiniques sur laquelle l'encre colorante de la couche portée par ladite au moins une région est destinée à être appliquée.

4. Procédé selon l'une quelconque des revendications précédentes, la couche d'encre colorante (4₁, ..., 4ₙ) comportant un corps huileux et/ou le substrat (2) étant en un matériau non absorbant, de préférence en matière plastique.

5. Procédé selon l'une quelconque des revendications précédentes, les couches d'encre colorante (4₁, ..., 4ₙ) situées dans les différentes régions différant au moins de par leur couleur et/ou
au moins l'une des couches d'encre colorante (4₁, ..., 4ₙ) étant imprimée de manière à former un motif reproduisant un aspect d'hétérogénéités de relief et/ou de couleur de la peau.

6. Procédé selon l'une quelconque des revendications précédentes, l'impression de ladite au moins une couches (4₁, ..., 4ₙ) étant effectuée en quadrichromie ou avec plus de quatre encres de couleurs différentes, avec de préférence une résolution comprise entre 16 dpi et 1600 dpi.

7. Procédé selon l'une quelconque des revendications précédentes, l'encre étant aqueuse et/ou
l'encre étant pulvérulente.

8. Procédé de préparation d'un dispositif (1) selon l'une quelconque des revendications précédentes, l'imprimante étant une imprimante laser à four désactivé.

9. Procédé selon l'une quelconque des revendications précédentes, comportant, avant l'impression, une étape de simulation (34) de l'aspect des matières kératiniques revêtues d'une ou plusieurs des encres colorantes à imprimer, la simulation (34) de l'aspect des matières kératiniques revêtues étant affichée sur un écran d'un appareil.

10. Procédé selon l'une quelconque des revendications précédentes, comportant une étape d'enregistrement (35), sur un support de stockage de données, d'une information relative au dispositif préparé, l'information étant de préférence relative à la nature des encres colorantes présentes sur le dispositif préparé et/ou à la nature des matières kératiniques destinées à être revêtues par tout ou partie des encres colorantes du dispositif préparé.

11. Procédé selon l'une quelconque des revendications précédentes, le choix de l'encre colorante de ladite couche (4₁, ..., 4ₙ) étant réalisé en fonction d'une information relative à l'aspect des matières kératiniques à maquiller, notamment après réalisation d'une acquisition d'au moins une image des matières kératiniques à maquiller et/ou réalisation d'au moins une mesure d'au moins une caractéristique optique de ces matières kératiniques.

12. Procédé selon l'une quelconque des revendications précédentes, ladite au moins une imprimante effectuant un premier passage d'impression permettant d'obtenir une première fraction de la couche d'encre colorante puis au moins un deuxième passage d'impression permettant d'obtenir une deuxième fraction de la couche d' encre colorante superposée à la première fraction.

13. Procédé selon l'une quelconque des revendications précédentes, comportant une étape de récupération d'une information relative à la nature de la couche d'encre colorante (4₁, ..., 4ₙ) stockée sur un support informatique, l'impression étant effectuée en fonction de cette information.

## Patentansprüche

1. Verfahren zur Herstellung einer Kosmetikanordnung (60), die innerhalb ein und derselben Packung eine Schminkvorrichtung (1) aufweist, die ein Substrat (2) aufweist, das aus einem starren oder halbstarren Material hergestellt ist und eine Druckfläche (3) definiert, die wenigstens drei Bereiche (3₁, ..., 3ₙ) aufweist, die jeweils mit wenigstens einer unterschiedlichen Schicht aus Färbemittel (4₁, ..., 4ₙ) bedruckt ist, das dazu bestimmt ist, auf die menschlichen keratinischen Materialien aufgetragen zu werden, von denen eine Schicht ein oder mehrere Färbemittel aufweisen kann,
wobei sich die Färbemittelschichten aufgrund der chemischen Beschaffenheit des oder der Färbemittel, aus denen sie zusammengesetzt sind, oder aufgrund der relativen Proportionen jedes Färbemittels, aus denen sie zusammengesetzt sind, unterscheiden, wobei die wenigstens drei Färbemittel
- durch Aufdruck auf die Druckfläche (3) durch wenigstens einen Digitaldrucker aufgebracht wurden
- nicht von einem Haftmittel bedeckt sind, und
- nach Auftragung auf die keratinischen Materialien wenigstens einen sichtbaren optischen Effekt aus Farbe und/oder Brillanz bewirken, wobei die Vorrichtung (1) insbesondere wenigstens drei, vorzugsweise wenigstens vier Bereiche (3₁, ..., 3ₙ) aufweist und
wobei das Verfahren den Schritt aufweist, der darin besteht, mithilfe wenigstens eines Druckers, insbesondere eines Digitaldruckers, wenigstens eine Schicht aus Kosmetikfärbemittel (4₁, ..., 4ₙ) auf wenigstens drei Bereiche (3₁, ..., 3ₙ) einer Druckfläche (3) eines Substrats (2) zu drucken, insbesondere eine unterschiedliche Färbemittelschicht auf jeden Bereich einer Mehrzahl von Bereichen zu drucken,
wobei das Verfahren **dadurch gekennzeichnet ist, dass** die Vorrichtung einen Applikator für die Auftragung aller oder einiger der Färbemittel der Vorrichtung auf die menschlichen keratinischen Materialien aufweist, und dadurch, dass der Drucker ein Tintenstrahldrucker oder ein Laserdrucker ist.

2. Verfahren nach Anspruch 1, wobei die wenigstens eine Schicht (4₁, ..., 4ₙ) ein Färbemittel oder mehrere Färbemittel aufweist, die dazu bestimmt sind, auf die menschlichen keratinischen Materialien aufgetragen zu werden, und geeignet sind, durch Auftragung auf die keratinischen Materialien ohne Hinzufügung einer Dritt-Fluidkomponente ein Make-up zu erzeugen.

3. Verfahren nach einem der vorhergehenden Ansprüche, wobei wenigstens zwei Färbemittelschichten (4₁, ..., 4ₙ) einen Verlauf ein und desselben optischen Effekts, vorzugsweise Farbe, entlang eines Wegs (C) erzeugen, der die Bereiche (3₁, ..., 3ₙ) verbindet, und/oder der wenigstens eine Bereich (3₁, ..., 3ₙ) einem Indikator (20₁, ..., 20ₙ) zugeordnet ist, der vorzugsweise vom Substrat (2) getragen wird, der es ermöglicht, eine Information über den Ort des Bereichs der keratinischen Materialien bereitzustellen, auf den das Färbemittel der Schicht, die von dem wenigstens einen Bereich getragen wird, aufgetragen werden soll.

4. Verfahren nach einem der vorhergehenden Ansprüche, wobei die Färbemittelschicht (4₁, ..., 4ₙ) einen Ölkörper aufweist und/oder das Substrat (2) aus einem nicht absorbierenden Material ist, vorzugsweise aus Kunststoff.

5. Verfahren nach einem der vorhergehenden Ansprüche, wobei die Färbemittelschichten (4₁, ..., 4ₙ), die sich in den verschiedenen Bereichen befinden, sich wenigstens durch ihre Farbe unterscheiden und/oder wenigstens eine der Färbemittelschichten (4₁, ..., 4ₙ) so gedruckt ist, dass sie ein Muster bildet, das einen Aspekt von Heterogenitäten des Reliefs und/oder der Farbe der Haut wiedergibt.

6. Verfahren nach einem der vorhergehenden Ansprüche, wobei der Druck der wenigstens einen Schicht (4₁, ..., 4ₙ) im Vierfarbdruck oder mit mehr als vier Färbemitteln verschiedener Farben durchgeführt wird, vorzugsweise mit einer Auflösung zwischen 16 dpi und 1600 dpi.

7. Verfahren nach einem der vorhergehenden Ansprüche, wobei das Färbemittel wässrig und/oder das Färbemittel pulverartig ist.

8. Verfahren zur Herstellung einer Vorrichtung (1) nach einem der vorhergehenden Ansprüche, wobei der Drucker ein Laserdrucker mit deaktivierter Fixiereinheit ist.

9. Verfahren nach einem der vorhergehenden Ansprüche, aufweisend, vor dem Druck, einen Schritt des Simulierens (34) des Erscheinungsbilds der keratinischen Materialien, die mit einem oder mehreren der aufzudruckenden Färbemittel bedeckt sind, wobei die Simulation (34) des Erscheinungsbilds der bedeckten keratinischen Materialien auf einem Bildschirm eines Geräts angezeigt wird.

10. Verfahren nach einem der vorhergehenden Ansprüche, aufweisend einen Schritt des Speicherns (35) einer Information in Bezug auf die hergestellte Vorrichtung auf einem Datenspeichermedium, wobei sich die Information vorzugsweise auf die Beschaffenheit der Färbemittel, die auf der hergestellten Vorrichtung vorhanden sind, und/oder auf die Beschaffenheit der keratinischen Materialien, die von allen oder einigen der Färbemittel der hergestellten Vorrichtung bedeckt sein sollen, bezieht.

11. Verfahren nach einem der vorhergehenden Ansprüche, wobei die Wahl des Färbemittels der Schicht (4₁, ..., 4ₙ) in Abhängigkeit von einer Information in Bezug auf das Erscheinungsbild der zu schminkenden keratinischen Materialien vorgenommen wird, insbesondere nach Durchführung einer Erfassung wenigstens eines Bilds der zu schminkenden keratinischen Materialien und/oder Durchführung wenigstens einer Messung wenigstens eines optischen Merkmals dieser keratinischen Materialien.

12. Verfahren nach einem der vorhergehenden Ansprüche, wobei der wenigstens eine Drucker einen ersten Druckdurchlauf, der es ermöglicht, einen ersten Anteil der Färbemittelschicht zu erhalten, dann wenigstens einen zweiten Druckdurchlauf durchführt, der es ermöglicht, einen zweiten Anteil der Färbemittelschicht zu erhalten, der den ersten Anteil überlagert.

13. Verfahren nach einem der vorhergehenden Ansprüche, aufweisend einen Schritt des Abrufens einer Information in Bezug auf die Beschaffenheit der Färbemittelschicht (4₁, ..., 4ₙ), die auf einem Datenträger gespeichert ist, wobei der Druck in Abhängigkeit von dieser Information durchgeführt wird.

## Claims

1. Process for preparing a cosmetic assembly (60) comprising, in the same packaging, a makeup device (1) comprising a substrate (2) made of a rigid or semi-rigid material and defining a printing surface (3) having at least three regions (3₁, ..., 3ₙ) each printed with at least one layer of different colouring ink (4₁, ..., 4ₙ) intended to be applied to human keratin materials, one layer of which may comprise one or more inks, the layers of ink being different by virtue of the chemical nature of the ink(s) of which they are composed or by virtue of the relative proportions of each ink of which they are composed, said at least three colouring inks
- having been deposited by printing on the printing surface (3) by at least one digital printer,
- not being covered with an adhesive, and
- producing, after application to the keratin materials, at least one visible optical effect among colour and/or sheen, the device (1) comprising in particular at least three, preferably at least four, regions (3₁, ..., 3ₙ) and
the process comprising the step consisting in printing, using at least one printer, in particular digital printer, at least one layer of cosmetic colouring ink (4₁, ..., 4ₙ) on at least three regions (3₁, ..., 3ₙ) of a printing surface (3) of a substrate (2), in particular printing a layer of different colouring ink on each region of a plurality of regions,
the process being **characterized in that**
the device comprises an applicator for the application of all or some of the colouring inks of the device to human keratin materials, and **in that**
the printer is an inkjet printer or a laser printer.

2. Process according to Claim 1, said at least one layer (4₁, ..., 4ₙ) comprising an ink or several inks intended to be applied to human keratin materials and capable of producing a makeup result by application to the keratin materials without addition of an intermediary fluid compound.

3. Process according to either one of the preceding claims, at least two layers of colouring ink (4₁, ..., 4ₙ) producing a gradation of the same optical effect, preferably the colour, along a path (C) connecting the regions (3₁, ..., 3ₙ) and/or said at least one region (3₁, ..., 3ₙ) being associated with an indicator (20₁, ..., 20ₙ), preferably borne by the substrate (2), making it possible to provide information on the location of the area of the keratin materials to which the colouring ink of the layer borne by said at least one region is intended to be applied.

4. Process according to any one of the preceding claims, the layer of colouring ink (4₁, ..., 4ₙ) comprising an oily substance and/or the substrate (2) being made of a non-absorbent material, preferably made of plastic.

5. Process according to any one of the preceding claims, the layers of colouring ink (4₁, ..., 4ₙ) located in the various regions differing at least by virtue of their colour and/or
at least one of the layers of colouring ink (4₁, ..., 4ₙ) being printed so as to form a pattern reproducing an appearance of relief and/or colour heterogeneities of the skin.

6. Process according to any one of the preceding claims, the printing of said at least one layer (4₁, ..., 4ₙ) being carried out by four-colour printing or with more than four inks of different colours, preferably with a resolution of between 16 dpi and 1600 dpi.

7. Process according to any one of the preceding claims, the ink being aqueous and/or
the ink being pulverulent.

8. Process for preparing a device (1) according to any one of the preceding claims, the printer being a laser printer having a deactivated fuser.

9. Process according to any one of the preceding claims, comprising, before printing, a step of simulating (34) the appearance of the keratin materials coated with one or more of the colouring inks to be printed, the simulation (34) of the appearance of the keratin materials coated being displayed on a screen of a machine.

10. Process according to any one of the preceding claims, comprising a step of recording (35), on a data storage medium, information relating to the device prepared, the information preferably relating to the nature of the colouring inks present on the device prepared and/or to the nature of the keratin materials intended to be coated with all or some of the colouring inks of the device prepared.

11. Process according to any one of the preceding claims, the choice of the colouring ink of said layer (4₁, ..., 4ₙ) being made according to information relating to the appearance of the keratin materials to be made up, in particular after performing an acquisition of at least one image of the keratin materials to be made up and/or performing at least one measurement of at least one optical characteristic of these keratin materials.

12. Process according to any one of the preceding claims, said at least one printer performing a first round of printing making it possible to obtain a first fraction of the layer of colouring ink and then at least one second round of printing making it possible to obtain a second fraction of the layer of colouring ink superposed on the first fraction.

13. Process according to any one of the preceding claims, comprising a step of recovering information relating to the nature of the layer of colouring ink (4₁, ..., 4ₙ) stored on a computer medium, the printing being carried out according to this information.
